(19) Europäisches Patentamt
European Patent Office
Office européen des brevets

(11) **EP 4 293 041 A1**

(12) **EUROPEAN PATENT APPLICATION**
published in accordance with Art. 153(4) EPC

(43) Date of publication:
**20.12.2023 Bulletin 2023/51**

(21) Application number: **22752339.6**

(22) Date of filing: **10.02.2022**

(51) International Patent Classification (IPC):
*C07K 14/715* (2006.01)  *A61K 35/17* (2015.01)
*C12N 5/10* (2006.01)  *A61P 35/00* (2006.01)

(52) Cooperative Patent Classification (CPC):
**A61K 35/17; A61P 35/00; C07K 14/715; C12N 5/10**

(86) International application number:
**PCT/CN2022/075873**

(87) International publication number:
**WO 2022/171179 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.02.2021 CN 202110184892**

(71) Applicant: **Cure Genetics Co., Limited**
**Suzhou, Jiangsu 215123 (CN)**

(72) Inventors:
• **HUANG, Zhuo**
**Suzhou, Jiangsu 215123 (CN)**
• **LIN, Yanni**
**Suzhou, Jiangsu 215123 (CN)**
• **ZHAO, Xun**
**Suzhou, Jiangsu 215123 (CN)**
• **ZHENG, Xiaocui**
**Suzhou, Jiangsu 215123 (CN)**

(74) Representative: **Bardehle Pagenberg Partnerschaft mbB**
**Patentanwälte Rechtsanwälte**
**Prinzregentenplatz 7**
**81675 München (DE)**

Remarks:
The complete document including Reference Table(s) and the Sequence Listing(s) can be downloaded from the EPO website

(54) **AMPLIFICATION ENHANCER AND USE THEREOF**

(57) The present invention relates to a proliferation enhancer. The enhancer comprises a protein molecule capable of initiating the cellular STATS and/or STAT3 signalling pathway and comprising the intracellular domain, the transmembrane domain and the extracellular domain. The present invention further relates to a lymphocyte expressing the proliferation enhancer and the use thereof as an immunotherapy drug.

*FIG. 1*

**Description**

**Technical Field**

The present invention relates to a proliferation enhancer, in particular to a protein molecule comprising an intracellular domain capable of initiating the cellular STATS and/or STAT3 signalling pathway, a transmembrane domain and an extracellular domain, and a lymphocyte comprising the proliferation enhancer and the use thereof as an immunotherapy drug.

**Background Art**

Immune cell therapy, such as chimeric antigen receptor T cell (CAR-T) therapy, has shown good therapeutic effects in cancer treatment. Existing autologous CAR-T technology requires personalized cell preparation, which is limited by long production cycle, high cost and a lack of sufficient patient T cells in many cases. In universal immune cell therapy, such as universal CAR-T (UCAR-T) therapy, T cells are derived from healthy donors, and cells for patients are prepared in advance, which may realize the standardized and unified production process, may realize the timeliness and standardization of administration in clinical application, and has low cost. However, the survival and proliferation ability of allogeneic immune cells in the body is limited. On one hand, there is rejection between different individuals, and on the other hand, the low cytokine environment in the body is unfavourable for the survival and proliferation of universal CAR-T, which greatly limits the effectiveness of such therapy.

CN 109952309 discloses a constitutively active cytokine receptor for cell therapy, in particular a constitutively active IL-7 cytokine receptor C7R from transmembrane and intracellular domains of IL-7R$\alpha$ and an extracellular domain of CD34, which may promote the in vivo proliferation of T cells and be used for the treatment of medical conditions including cancers. However, in this solution, CD34 is very large, and a combination of CD34 and CAR may affect the expression efficiency and stability of CAR molecules in T cells, and also affect the preparation and large-scale production of products.

In addition, the use of T cells in universal CAR-T needs to solve the problem of two-way rejection between allogeneic cells. The current technologies for preparing universal CAR-T involve removing the expression of TCR complex of donor T cells, which may prevent donor T cells from recognizing and killing host cells, and also involve removing the expression of class I MHC molecules of donor T cells, which may eliminate the antigen presentation by donor cells and evade the recognition and killing by host T cells (CN 106103475 A). However, the lack of class I MHC molecules may initiate receptor NK cell-mediated killing.

Therefore, how genetically engineered immune cells can improve the proliferation and survival ability and enhance the lasting anti-tumour activity while overcoming allogeneic transplantation rejection is a long-standing unmet need in the field of cell therapy.

**Summary of the Invention**

In order to improve the proliferation ability of lymphocytes, the present invention provides a proliferation enhancer. The proliferation enhancer comprises the following components: a) one or more cytokine receptor intracellular domains capable of initiating a cellular STATS and/or STAT3 signalling pathway; b) a transmembrane domain; c) one or more extracellular domains that are extracellular domains of DAP12 or variants thereof; wherein the transmembrane domain and the extracellular domain comprise a structure that promotes the homodimerization of the proliferation enhancer.

The present invention further provides a genetically engineered cell, characterized in that on the surface of the cell, the proliferation enhancer of the present invention is expressed.

In a specific embodiment, the present invention provides a CAR-T cell expressing the proliferation enhancer of the present invention. In the absence of cytokines (such as IL-2), the proliferation ability of CAR-T cells edited by this gene is improved.

In a specific embodiment, the present invention further provides a CAR-T cell simultaneous expressing the proliferation enhancer of the present invention and a chimeric single chain molecule; and the chimeric single chain molecule comprises: (a) a presenting peptide fragment, (b) a B2M protein, and (c) a linker sequence for linking the aforementioned fragments (a) and (b), wherein the chimeric single chain molecule forms a complex on the cell membrane with a heavy chain molecule of MHC or an MHC analogue, and the presenting peptide fragment is a polypeptide sequence comprising 5-30 amino acids.

The present invention discloses the technical solutions as shown by the following serial numbers:

    1. A proliferation enhancer, comprising the following components: a) one or more cytokine receptor intracellular domains capable of initiating a cellular STAT5 and/or STAT3 signalling pathway; b) a transmembrane domain; c) one or more extracellular domains that are extracellular domains of DAP12 or variants thereof; wherein the transmembrane domain and the extracellular domain comprise a structure that promotes the homodimerization of the proliferation enhancer.

2. The proliferation enhancer of claim 1, wherein the receptor intracellular domain is from an IL-7 cytokine receptor α, an IL-21 cytokine receptor α, an IL-23 cytokine receptor α, an IL-12 cytokine receptor α, CD122, or a combination thereof.

3. The proliferation enhancer of claim 1, wherein the receptor intracellular domain is a sequence of amino acids at positions 47-241 of SEQ ID NO: 3, amino acids at positions 47-245 of SEQ ID NO: 4, amino acids at positions 47-251 of SEQ ID NO: 5 or amino acids at positions 47-266 of SEQ ID NO: 6.

4. The proliferation enhancer of any one of the preceding claims, wherein the transmembrane domain is an endogenous transmembrane domain of component a) one or more cytokine receptor intracellular domains, or a variant of the endogenous transmembrane domain.

5. The proliferation enhancer of any one of the preceding claims, wherein the transmembrane domain is a transmembrane domain of an IL-7 cytokine receptor α or a variant thereof, or the transmembrane domain is a transmembrane domain of DAP12 or a variant thereof.

6. The proliferation enhancer of any one of the preceding claims, wherein the transmembrane domain comprises at least one cysteine.

7. The proliferation enhancer of any one of the preceding claims, wherein the transmembrane domain is a sequence having a length of 21-33 amino acids.

8. The proliferation enhancer of claim 7, wherein the transmembrane domain is a sequence of amino acids at positions 19-46 of SEQ ID NO: 3, or the transmembrane domain is SEQ ID NO: 11, or the transmembrane domain is a sequence of amino acids at positions 20-40 of SEQ ID NO: 15.

9. The proliferation enhancer of any one of the preceding claims, wherein the extracellular domain is an extracellular domain of DAP12 having a sequence as shown in amino acids at positions 7-18 of SEQ ID NO: 3, or a polypeptide sequence comprising an extracellular domain of DAP12, or a variant of an extracellular domain of DAP12 which is derived by performing substitution, deletion or addition of one or several amino acids on an extracellular domain of DAP12.

10. The proliferation enhancer of claim 9, wherein the extracellular domain has a length of not more than 130 amino acids; preferably not more than 120 amino acids; preferably not more than 40 amino acids; preferably not more than 20 amino acids; preferably, the extracellular domain has a length of 10-40 amino acids or 12-20 amino acids.

11. A nucleic acid sequence expressing the proliferation enhancer of any one of the preceding claims.

12. A genetically engineered cell, characterized in that on the surface of the cell, the proliferation enhancer of any one of claims 1-10 is expressed.

13. The cell of claim 12, wherein the cell is an immune cell, and further the immune cell is preferably a lymphocyte.

14. The cell of claim 13, wherein the lymphocyte is a T cell, an NKT cell, a γδT cell, a mucosa associated invariant T cell, an NK cell or a B cell.

15. The cell of any one of claims 12-14, wherein the surface of the cell lacks at least one endogenously expressed MHC molecule or MHC molecule analogue.

16. The cell of any one of claims 12-15, wherein at least one gene encoding a component of an endogenous T cell receptor (TCR) in the cell is inactivated.

17. The cell of any one of claims 12-16, wherein the T cell is a CAR-T cell or a TCR-T cell.

18. The cell of any one of claims 12-17, wherein the cell has a nucleic acid sequence expressing a chimeric single chain molecule, and the chimeric single chain molecule comprises: (a) a presenting peptide fragment, (b) a B2M protein, and (c) a linker sequence for linking the aforementioned fragments (a) and (b); wherein, the chimeric single chain molecule forms a complex on the cell membrane with a heavy chain molecule of MHC or an MHC analogue; and the presenting peptide fragment is a polypeptide sequence of 5-30 amino acids.

19. The cell of claim 18, wherein the chimeric single chain molecule sequentially comprises, from the N-terminus to the C-terminus, presenting peptide fragment-linker sequence-B2M protein, or B2M protein-linker sequence-presenting peptide fragment.

20. The cell of claim 18 or 19, wherein the heavy chain molecule of MHC is a heavy chain molecule endogenously expressed in a cell and selected from a classical class I MHC molecule, preferably a heavy chain molecule of HLA-A, HLA-B or HLA-C; or the heavy chain molecule of MHC is a heavy chain molecule endogenously expressed in a cell and selected from a non-classical class I MHC molecule, preferably a heavy chain molecule of HLA-E, HLA-F or HLA-G; or the heavy chain molecule of MHC is a heavy chain molecule endogenously expressed in a cell and selected from other non-classical class I MHC molecules, preferably a heavy chain molecule of CD1 or MR1 or LTL18.

21. The cell of any one of claims 18-20, wherein the presenting peptide fragment is derived from a signal peptide of a class I MHC molecule.

22. The cell of any one of claims 17-21, wherein the cell lacks an endogenously expressed MHC molecule, and lacks an endogenously expressed T cell receptor.

23. The cell of claim 22, wherein the chimeric single chain molecule expressed by the cell forms a complex on the cell membrane with a heavy chain molecule of MHC or an MHC analogue.

24. Use of the cell of any one of claims 12-23 in the preparation of a drug for treating a cancer.

25. A method for treating a cancer, comprising: administering to an individual the genetically engineered cell of claims 12-23.

26. The use of claim 24 and the method of claim 25, wherein the cancer is breast cancer, prostate cancer, lung cancer, brain cancer, colon cancer, head and neck cancer, skin cancer, ovarian cancer, endometrial cancer, cervical cancer, kidney cancer, lung cancer, gastric cancer, small intestine cancer, liver cancer, pancreatic cancer, gallbladder cancer, carcinoma of bile duct, oesophageal cancer, salivary adenocarcinoma or thyroid cancer.

## Detailed Description of the Invention

### Definitions

Cytokine

Cytokines refer to cell signalling molecules for regulating the immune system's response to inflammations and infections and helping with intercellular communication in immune response.

Intracellular domain

A proliferation enhancer comprises one or more intracellular domains. The intracellular domain may be from a molecule identical to a transmembrane domain, but this is not the case in other instances. In a specific embodiment, the intracellular domain comprises STATS and STAT3 binding sites and thus may play a role in the STATS and STAT3 signalling pathways. Intracellular domains of immunostimulating cytokines of receptors useful in the content of the present invention are from, for example, an IL-7 cytokine receptor $\alpha$, an IL-21 cytokine receptor $\alpha$, an IL-23 cytokine receptor $\alpha$, an IL-12 cytokine receptor $\alpha$, CD122 or a combination thereof.

In a particular embodiment, the intracellular domain comprises an intracellular domain from an IL7 receptor $\alpha$ chain; preferably amino acids at positions 47-241 of SEQ ID NO: 3, amino acids at position 47-245 of SEQ ID NO: 4, amino acids at positions 47-251 of SEQ ID NO: 5, and amino acids at positions 47-266 of SEQ ID NO: 6.

In some embodiments, the intracellular domain has a length of 70-250 amino acids, 70-200 amino acids, 80-100 amino acids, 90-250 amino acids, 90-100 amino acids, 100-250 amino acids, 100-125 amino acids, 125-150 amino acids, 150-175 amino acids, 175-200 amino acids, 200-225 amino acids, etc. In a specific embodiment, these fragments having these lengths retain STATS and STAT3 signalling activity.

Transmembrane domain

The proliferation enhancer of the present invention comprises a transmembrane domain operably linked to an extracellular domain and an intracellular domain. The transmembrane domain may be from a natural molecule identical to the intracellular domain operably linked to the transmembrane domain, or may be from different natural molecule. In a specific embodiment, the transmembrane domain comprises one or more structures that initiate or promote homodimerization, such as a disulphide bond.

In some cases, the transmembrane domain uses mutations identified in patients with tumours as gain-of-function mutations in the transmembrane domain. In at least some cases, the mutant form includes a cysteine insertion that induces the formation of a disulphide bond in the transmembrane domain. However, in other embodiments, one or more mutations lack cysteine insertion. For example, a transmembrane domain derivative may be used, and does not have a cysteine insertion (and therefore does not have a disulphide bond) but still emits a signal and is constitutionally active, for example, a mutation causes a conformational change in a transmembrane domain compared to the natural form of the transmembrane domain, thereby allowing the induction of signalling.

In a specific embodiment, the transmembrane domain is from an IL-7R$\alpha$ receptor, and the mutation in the transmembrane domain is in a sequence PILLTISILSFFSVALLVILACVLW (SEQ ID NO: 11). In another specific embodiment, the transmembrane domain is a transmembrane domain from DAP12 or a variant thereof, for example, a transmembrane region in an amino acid sequence of DAP12 with a Genbank number of NP_003323.1 (amino acids at positions 20-40 of SEQ ID NO: 15). In some embodiments, the mutation is or comprises the insertion of one or more cysteines and/or one or more prolines into SEQ ID NO: 11, or an amino acid sequence of amino acids at positions 20-40 of SEQ ID NO: 15, wherein the mutation enables or promotes the homodimerization of a receptor. In some cases, the mutation comprises inserting a trimer peptide of cysteine, proline and threonine (CPT) into a transmembrane domain. This mutation confers the formation of a disulphide bond between the -SH (mercapto) groups of the cysteine residues of two molecules (as examples, two IL7RP2 receptor $\alpha$ chains), allowing the formation of a homodimer between them (In a specific embodiment, proline next to the cysteine helps to twist the homodimer in the correct direction). In a specific embodiment, threonine in CPT insertion is not threonine but another amino acid, and in at least particular cases, the other amino acid is or is not cysteine or proline. In embodiments in which one or more amino acids are inserted into SEQ ID NO: 11 or amino acids at positions 20-40 of SEQ ID NO: 15 for use in a receptor, the insertion may be located between any two amino acids of SEQ ID NO: 11 or amino acids at positions 20-40 of SEQ ID NO: 15. In a particular embodiment, the insertion is located after the first, second, third, fourth, fifth, sixth, seventh, eighth, ninth, tenth, eleventh, twelfth, thirteenth, fourteenth, fifteenth, sixteenth, seventeenth, eighteenth, nineteenth, twentieth, twenty-first, twenty-second, twenty-third or twenty-fourth amino acid in SEQ ID NO: 11.

In a specific embodiment, known disclosed transmembrane domains may be used, for example, the transmembrane domain disclosed in Chinese patent CN 201780065147.0 (referred to as TM sequence in the patent).

Extracellular domain

The extracellular domain of the proliferation enhancer of the present invention is from an extracellular domain of DAP12 and corresponds to amino acids at positions 7-18 of SEQ ID NO: 3, and SEQ ID NOs: 3, 4, 5 and 6 have the same extracellular domain.

DAP12 gene is a transmembrane receptor, is widely present on the surface of natural killer cells, granulocytes and monocytes/macrophages, and plays an important role in transmitting active signals. Transmembrane protein DAP12 consists of three parts, a shorter extracellular region, a single-transmembrane region and an intracellular region containing an immunoreceptor tyrosine-based activation motif (ITAM). DAP12 may form a homodimer containing a disulphide bond.

In a specific embodiment, the extracellular domain of the present invention is an extracellular domain of DAP12 or a variant thereof. The variant of the extracellular domain of DAP12 is a polypeptide sequence comprising amino acids at positions 7-18 of SEQ ID NO: 3, for example, a fusion polypeptide formed by binding the extracellular domain of DAP12 to other functional fragments. The variant of the extracellular domain of DAP12 may also be an extracellular domain of DAP12 which is derivatized by performing substitution, deletion or addition of one or several amino acids on amino acids at positions 7-18 of SEQ ID NO: 3,

wherein the extracellular domain has a length of not more than 130 amino acids; preferably not more than 120 amino acids; preferably not more than 40 amino acids; preferably not more than 20 amino acids; preferably, the extracellular domain has a length of 30-120 amino acids, 30-100 amino acids, 30-90 amino acids, 30-70 amino acids, 30-60 amino acids, 25-60 amino acids, 30-50 amino acids, 35-45 amino acids, 36-42 amino acids, 38-40 amino acids, 39-40 amino acids or 39 amino acids, 10-40 amino acids, 12-20 amino acids, or 12 amino acids.

Variant

The variant of the present invention refers to a sequence having at least 80%, 83%, 85%, 86%, 87%, 88%, 89%, 90%, 91%, 92%, 93%, 94%, 95%, 96%, 97%, 98%, 99% or 100% identity to the sequences such as an extracellular domain of DAP12 and a transmembrane domain of an IL-7 cytokine receptor $\alpha$, and having the same or similar functions; or a protein sequence having the same or similar functions and obtained by the deletion, substitution or addition of one or several amino acids compared with the sequences; preferably, the variant has the deletion, substitution or addition of 10, 9, 8, 7, 6, 5, 4, 3, 2 or 1 amino acid compared with the sequences.

The correlation between two amino acid sequences is described by the parameter "identity".

In term of the present invention, the degree of identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends in Genetics 16: 276-277), preferably version 3.0.0 or higher. The optional parameters used are gap penalty of 10, gap extension penalty of 0.5, and substitution matrix EBLOSUM62 (EMBOSS version of BLOSUM62). The output result marked as "longest identity" by Needle (obtained using the - nobrief option) is used as the percentage identity, and is calculated as follows:

$$(identical\ residues \times 100)/(alignment\ length - total\ number\ of\ gaps\ in\ alignment).$$

Proliferation enhancer

The proliferation enhancer of the present invention is a constitutively active cytokine receptor, wherein the cytokine receptor has no ligand requirement, and the transmembrane domain and/or intracellular domain component of the cytokine receptor is configured to transmit an activation signal in the absence of receiving a corresponding signal from the extracellular domain of the cytokine receptor. The enhancer of the present invention is present in the form of homodimerization, so that the extracellular domain remains in a state of transmitting an activation signal to the intracellular signalling pathway.

In a specific embodiment, the proliferation enhancer has an amino acid sequence of Ev1-5 as shown in SEQ ID NO: 3, 4, 5, 6 or 15. Ev1-5 represents one sequence of proliferation enhancers Ev1, Ev2, Ev3, Ev4 and Ev5.

Class I MHC molecule

Class I MHC molecule, also referred to as class I major histocompatibility complex, is a heterodimer glycoprotein consisting of two peptide chains linked by a non-covalent bond; wherein one is referred to as a heavy chain, which is polymorphic in structure, and the other is a light chain or referred to as $\beta2$ microglobulin (B2M). Functionally, class I MHC molecules may present polypeptides that are degraded intracellularly and are not self-proteins, thus activating the immune system. Human MHC molecules are also referred to as HLA molecules. Classical class I MHC molecules (also called MHC-Ia) include HLA-A, HLA-B and HLA-C. Non-classical class I MHC molecules (also called MHC-Ia) include HLA-E, HLA-F and HLA-G.

MHC-like molecule

MHC-like molecules, also called MHC molecule analogues in the present patent, refer to complexes that do not belong to class I MHC molecules, but have a structure similar to that of class I MHC molecules, and are also formed by the non-covalent binding of heavy chains to B2M proteins. For example, non-classical MHC-like molecules include but are not limited to CD1 and MR1. As another example, LTL18 protein derived from human cytomegalovirus (HCMV), has homology with a heavy chain of class I MHC molecule, and binds to B2M to form a complex expressed on the cell membrane, which may enable the cell to evade the killing by NK cells.

Chimeric single chain molecule (CM)

The chimeric single chain molecule (also referred to as "chimeric molecule"), expressed on the surface of a cell, of the present invention sequentially comprises, from the N-terminus to the C-terminus: presenting peptide fragment-linker sequence-B2M protein, or B2M protein-linker sequence-presenting peptide fragment. Chimeric single chain molecule (CM) has an effect of inhibiting rejection.

T cells in universal CAR-T are from healthy donors and may be prepared in advance for use by any patient, but needs to solve the problem of two-way rejection between allogeneic cells. The current technologies for preparing universal CAR-T involve removing the expression of TCR complex of donor T cells, which may prevent donor T cells from recognizing and killing host cells, and also involve removing the expression of class I MHC molecules of donor T cells, which may eliminate the antigen presentation by donor cells and evade the recognition and killing by host T cells (CN 106103475 A). However, the lack of class I MHC molecules may initiate receptor NK cell-mediated killing. According to the present invention, a specific type of a chimeric single chain molecule formed by coupling a presenting peptide (such as a presenting peptide specifically binding to HLA-E/HLA-G/HLA-C) and B2M is expressed in an engineered universal immune cell, which may avoid the killing by allogeneic (or autologous) NK cells and other immune cells, thereby improving the compatibility of allogeneic transplantation.

In a specific embodiment, the chimeric single chain molecule is: membrane localization signal peptide-presenting peptide fragment-$(G_4S)_3$-B2M mature protein, in which the membrane localization signal peptide is removed before a chimeric molecule is located on the cell membrane.

Presenting peptide fragment

The "presenting peptide fragment" is also referred to as "presenting peptide" and "presenting polypeptide" in the present invention. Presenting peptide fragments are a class of short amino acid peptides, which may stably bind to the antigen binding groove of MHC molecules to form stable MHC complexes. Most of the presenting peptide fragments have a length of 7-30 amino acids, preferably 7-17 amino acids, more preferably 7-12 amino acids, and most preferably 8-10 amino acids. Only when a presenting peptide fragment matches with the antigen binding groove of an MHC molecule, "B2M protein coupled with a presenting peptide" (i.e., a chimeric single chain molecule) and a heavy chain molecule of MHC may form a complex, which is stably presented on the cell membrane and then recognized by immune cells. Different MHC molecules match with unique presenting peptide fragments.

In particular, according to different target HLA expressed, a specific peptide fragment matched with the heavy chain of a target HLA may be selected. For example, selecting a presenting peptide fragment matched with the heavy chain of HLA-C may enable a complex molecule formed by a chimeric single chain molecule and the heavy chain of HLA-C to be stably present on the cell membrane; as another example, selecting a presenting peptide fragment matched with the heavy chain of HLA-E may enable a complex formed by a chimeric single chain molecule and the heavy chain of HLA-E to be stably present on the cell membrane. In particular, for a chimeric molecule capable of forming a complex with the heavy chain molecule of HLA-E, the presenting polypeptide is derived from a signal peptide of class I MHC molecules, such as a signal peptide of HLA-A2, HLA-B7, HLA-B15, HLA-Cw3, HLA-Cw7, HLA-G, HLA-F, etc.; a protein polypeptide derived from viruses, such as a protein polypeptide from CMV(UL40), EBV, HIV, etc.

In a specific embodiment, most of the presenting polypeptides derived from signal peptides of class I MHC molecules have a structure of the following general formula: VM (A/P) PRT (L/V) (V/L/I/F)L or V (T/A) (A/P) PRT (L/V) (V/L/I/F)L, which represents a peptide fragment of 9 amino acids, wherein "()" represents the same amino acid position, and symbol "/" represents "or".

Preferably, the signal peptide derived from class I MHC molecules is selected from one of the following sequences:

Val Met Ala Pro Arg Thr Leu Ile Leu,
Val Met Ala Pro Arg Thr Leu Val Leu,
Val Met Ala Pro Arg Thr Leu Phe Leu,
Val Met Ala Pro Arg Thr Leu Leu Leu,
Val Met Ala Pro Arg Thr Val Leu Leu, and
Val Thr Ala Pro Arg Thr Leu Val Leu.

Presenting peptide fragments to which HLA-E complexes may bind are also listed in the following documents: Celik et al., Immunogenetics, 2016, 68:29-41; Hannoun et al., Immunology Letters, 2018, 202:65-72; Rölle et al., Cell Rep, 2018., 14(8):1967-1976; Rölle et al., Front Immunol, 2018, 9:2410, for example, a peptide fragment of 10 amino acids: YLLPRRGPRL.

B2M

β-2 microglobulin, also referred to as B2M protein, is the light chain of class I MHC molecules, and is an integral part of class I MHC molecules. Human B2M protein consists of 119 amino acids (SEQ ID NO: 7) and has a molecular weight of 11800 Daltons. The lack of B2M molecule prevents a cell from normally expressing class I MHC molecules on the cell membrane. Such cell may be recognized and killed by NK cells.

Chimeric antigen receptor (CAR)

CAR (Chimeric Antigen Receptor) refers to a specific antigen receptor that simultaneously expressing some intracellular activation signals, and mainly consists of 3 parts: an extracellularly expressed antigen receptor, which is mainly from a single chain antibody variable region (scFv) or a single domain antibody/nanobody; a transmembrane structure linked to extracellular and intracellular structures; and an intracellular signalling domain or a domain binding to a signalling domain/complex, which mainly comprises a T cell activation signal and a co-stimulatory signal, wherein co-stimulatory molecules may comprise one or two co-stimulatory molecules, and common co-stimulatory molecules are CD28, CD137 (4-1BB), CD27, OX40, CD30, CD40, etc. The extracellular antigen receptor is mainly from a tumour related antigen and may be selected from the following antigens: CD19, CD20, CD22, CD123, CD33/IL3Ra, CD138, CD33, BCMA, CS1, C-Met, EGFRvIII, CEA, Her2, GD2, MAG3, GPC3, NY-ESO-1, etc.

Lack of at least one endogenously expressed MHC molecule or MHC molecule analogue on the surface of a cell

The lack of a certain molecule on the surface of a cell refers to a decrease in the detected level of the molecule on the surface of the cell compared to that under normal physiological conditions. For example, on the surface of a cell, a decrease in the expression of the molecule, or the expression of the molecule being lower than the detection limit is detected using an antibody specific to the molecule; or after the cell membrane is isolated, the molecule cannot be detected using Western blot technique, or a decrease in the expression of the molecule is detected. Endogenously expressed molecules refer to molecules expressed by cells without modification and transformation. For example, in one embodiment, when cells are not treated by modifications such as virus transfection, gene editing, or RNA interference, molecules expressed by the unmodified cells are referred to as endogenously expressed molecules.

In a specific embodiment, the lack of at least one class I MHC molecule on the surface of a cell refers to the lack of one or more of molecules such as HLA-A/B/C/E/F/G; and the lack of at least one class I MHC molecule analogue on the surface of a cell refers to including but not limited to, the lack of one or more of molecules such as CD1, MR1 and UL18. If a cell has a lack of B2M molecule, the level of class I MHC molecules or MHC molecular analogues on the surface of the cell may decrease. In an embodiment, the lack of at least one endogenously expressed MHC molecule or MHC molecule analogue on the surface of a cell may be achieved by inactivating B2M or a corresponding heavy chain gene. Removing or partially removing the expression of class I MHC molecules of donor cells may reduce or eliminate the antigen presentation by donor cells and evade the recognition and killing by host T cells, which is particularly beneficial to improving the compatibility of allogeneic transplantation.

Immune cell

Immune cells are multiple types of cells that are produced and mature in the immune system and have immune response functions. The immune cell of the present patent may be one of or a mixture of more of lymphocyte, macrophage, stem cell, progenitor cell or immune effector cell.

Further, the lymphocyte may be one of or a mixture of more of T cell, NKT cell, $\gamma\delta$T cell, mucosa associated invariant T cell (MAIT cell), NK cell, B cell, tumour infiltrating lymphocyte (TIL) and innate lymphocyte.

In a specific embodiment, the T cell may also be a T cell (CAR-T cell) comprising a chimeric antigen receptor or a TCR-T cell.

Universal immune cells refer to immune cells that may be used for allogeneic transplantation and do not produce GvHD response or produce controllable GvHD response.

Medicinal use

Provided herein is a method for treating a cancer, comprising: administering to an individual suffering from the cancer an effective amount of the genetically engineered cell of the present invention, in particular administering CAR-T cells that express specificity to the cancer, wherein the CAR-T cells additionally express the proliferation enhancer of the present invention. In a more specific embodiment, the cancer is a solid tumour, including, for example, glioblastoma. In another specific embodiment, the CAR-T cell targets antigen GD2. In a more specific embodiment, the proliferation enhancer comprises a transmembrane domain derived from IL-7 receptor $\alpha$, wherein the transmembrane domain comprises one or more mutations that promote the homodimerization of a cytokine receptor.

The present invention further provides the use of a genetically engineered cell in the preparation of a drug for treating a cancer.

In the specific embodiment of any treatment method provided herein, the cancer is a spongioblastoma. In some specific embodiments of the method provided herein, the cancer is breast cancer, prostate cancer, lung cancer (for example, small cell lung cancer or non-small cell lung cancer), brain cancer, colon cancer, head and neck cancer, skin cancer (for example, melanoma), ovarian cancer, endometrial cancer, cervical cancer, kidney cancer, gastric cancer, small intestine cancer, liver cancer, pancreatic cancer, gallbladder cancer, carcinoma of bile duct, oesophageal cancer, salivary adenocarcinoma or thyroid cancer.

**Brief Description of the Drawings**

FIG. 1. Schematic diagram of expression vector.

FIG. 2. Schematic diagram of co-expression of Ev1-5 proteins and CM protein (Ev1-5 is located on the cell membrane and self-assembled into a dimer relying on an extracellular domain and a transmembrane domain, and CM and the heavy chain molecule of HLA-E are assembled into an HLA-E protein complex)

FIG. 3-1 to FIG. 3-3. Analysis of CAR expression efficiency and expression stability of primary T cells by flow cytometry.

FIG. 4. Dynamic analysis of CAR expression efficiency of primary T cells (Day 5, Day 8, Day 11).

FIG. 5-1 to FIG. 5-2. Detection of expression of pSTAT5 and pSTAT3 by flow cytometry.

FIG. 6. Proliferation of universal CAR-T cells in cytokine-free environment.

FIG. 7-1 to FIG. 7-2. Detection of gene editing efficiency and CAR expression efficiency of universal CAR-T cells by flow cytometry.

FIG. 8. Detection of HLA-E expression abundance of universal CAR-T cells.

FIG. 9-1 to FIG. 9-3. Detection of proliferation of universal CAR-T cells in PBMC with immunosuppressive environment from different sources.

**Detailed Description of Embodiments**

The present invention is further described in detail by the following examples. These examples are only exemplary and do not limit the scope of the present invention.

The meanings of the abbreviations are as follows: "h" refers to hour, "min" refers to minute, "s" refers to second, "ms" refers to millisecond, "d" refers to day, "$\mu$L" refers to microlitre, "mL" refers to millilitre, "L" refers to litre, "bp" refers to base pair, "mM" refers to millimole, and "$\mu$M" refers to micromole.

Materials

| Anti-FMC63 antibody (primary antibody) | Anti-mouse FMC63 (BioSwan Laboratories, Co., Ltd., R19PB-100) |
| --- | --- |
| Anti-HLA-ABC antibody | Anti-HLA-ABC-APC (R&D, FAB7098A) |
| Anti-CD3 antibody | Anti-CD3-FITC (BD, 555916) |
| Anti-HLA-E antibody | Anti-HLA-E-PE (Biolegend, 342604), Anti-HLA-E-APC (Biolegend, 342606) |
| Anti-B2M antibody | Anti-B2M-PE (BD, 551337) |
| Anti-HLA-ABC antibody | Anti-HLA-ABC-APC (R&D, FAB7098A) |
| Cell staining | Dye eFluorTM 670 (eBioscience, 65-0840-90) |

**Example 1: Production of universal CAR-T cells co-expressing proliferation enhancer and chimeric molecule (CM)**

Step 1: Construction of DNA expressing proliferation enhancer

DNA sequences expressing 5 proliferation enhancers as follows were synthesized, respectively, and a DNA sequence expressing a signal peptide that helps cell membrane localization was added to the 5' end of the following sequences. The signal peptide has an amino acid sequence of SEQ ID NO: 14.

Proliferation enhancer 1 (Ev1) has an amino acid sequence of SEQ ID NO: 3, wherein an intracellular domain is a sequence of amino acids at positions 47-241 of SEQ ID NO: 3, a transmembrane domain is a sequence of amino acids at positions 19-46 of SEQ ID NO: 3, and an extracellular domain is a sequence of amino acids at positions 7-18 of SEQ ID NO: 3.

Proliferation enhancer 2 (Ev2) has an amino acid sequence of SEQ ID NO: 4, wherein an intracellular domain is a sequence of amino acids at positions 47-245 of SEQ ID NO: 4, a transmembrane domain is a sequence of amino acids at positions 19-46 of SEQ ID NO: 4, and an extracellular domain is a sequence of amino acids at positions 7-18 of SEQ ID NO: 4.

Proliferation enhancer 3 (Ev3) has an amino acid sequence of SEQ ID NO: 5, wherein an intracellular domain is a sequence of amino acids at positions 47-251 of SEQ ID NO: 5, a transmembrane domain is a sequence of amino acids at positions 19-46 of SEQ ID NO: 5, and an extracellular domain is a sequence of amino acids at positions 7-18 of SEQ ID NO: 5.

Proliferation enhancer 4 (Ev4) has an amino acid sequence of SEQ ID NO: 6, wherein an intracellular domain is a sequence of amino acids at positions 47-266 of SEQ ID NO: 6, a transmembrane domain is a sequence of amino acids at positions 19-46 of SEQ ID NO: 6, and an extracellular domain is a sequence of amino acids at positions 7-18 of SEQ ID NO: 6.

Proliferation enhancer 5 (Ev5) has an amino acid sequence of SEQ ID NO: 15, wherein an intracellular domain is a sequence of amino acids at positions 44-242 of SEQ ID NO: 15, a transmembrane domain is a sequence of amino acids at positions 19-43 of SEQ ID NO: 15, and an extracellular domain is a sequence of amino acids at positions 7-18 of SEQ ID NO: 15.

Step 2: Method for constructing plasmids

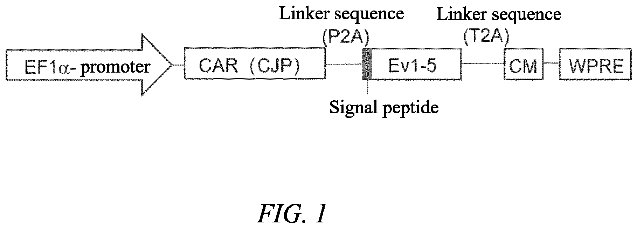

A lentiviral vector co-expressing a CAR molecule against CD19 (referred to as a CJP molecule), a proliferation enhancer (represented by Ev) and a chimeric molecule (represented by CM) was constructed. A viral expression plasmid (Addgene ID: #12252) was subjected to enzyme cleavage with BamHI/Sa1I for use as a backbone, and a core gene structure was formed by sequentially linking CJP-Ev-CM and using 2A self-cleaving peptides (P2A and T2A). CJP having a sequence of SEQ ID NO: 1 was respectively linked to one of five variants (represented by Ev1/2/3/4/5) of the proliferation enhancer by a GSG-P2A sequence, and was then linked to CM by a GSG-T2A sequence. A CJP-P2A-(Ev1-5)-T2A-CM sequence or a CJP-P2A-C7R-T2A-CM whole gene was synthesized and then linked into the digested viral expression backbone (FIG. 1). Eight viral expression plasmids containing CJP, a proliferation enhancer and a chimeric molecule were constructed:

> a target expression plasmid comprising CJP-P2A-Ev1-T2A-CM,
> a target expression plasmid comprising CJP-P2A-Ev2-T2A-CM,
> a target expression plasmid comprising CJP-P2A-Ev3-T2A-CM,
> a target expression plasmid comprising CJP-P2A-Ev4-T2A-CM,
> a target expression plasmid comprising CJP-P2A-Ev5-T2A-CM,
> a target expression plasmid comprising CJP-P2A-C7R-T2A-CM,
> a target expression plasmid comprising CJP,
> and a target expression plasmid comprising CJP-CM.

The sequence of C7R as a control is derived from patent CN 109952309, and has an amino acid sequence of SEQ ID NO: 2. Ev1 has an amino acid sequence of SEQ ID NO: 3, Ev2 has an amino acid sequence of SEQ ID NO: 4, Ev3 has an amino acid sequence of SEQ ID NO: 5, Ev4 has an amino acid sequence of SEQ ID NO: 6, and Ev5 has an amino acid sequence of SEQ ID NO: 15. CM has an amino acid sequence of SEQ ID NO: 7.

P2A and T2A are self-cleavage polypeptide linker sequences, and are common linker sequences simultaneous expressing two independent proteins in one transcript in a vector. During translation, a protein breaks apart at the ends of P2A and T2A sequences, so that two consecutive proteins linked by P2A and T2A separate and play a role separately. P2A has an amino acid sequence of ATNFSLLKQAGDVEENPGP (SEQ ID NO: 12), and T2A has an amino acid sequence of EGRGSLLTCGDVEENPGP (SEQ ID NO: 13).

Step 3: Method for preparing lentivirus

A three-plasmid system comprising a lentiviral target expression plasmid (the target expression plasmid constructed in step 2), packaging help plasmid psPAX2 (Addgene ID: #12260) and pMD2.G (Addgene ID: #12259) was used. Virus packaging was performed in HEK293T cells (purchased from Shanghai Institute of Biochemistry and Cell Biology, Chinese Academy of Sciences). The preparation process was as follows: HEK293T cells were resuscitated from cryopreserved working cells and cultured in a 10 cm culture dish with a DMEM medium (+ 10% FBS + 1% P/S) (Cellgro 10-013-CMR). The medium was changed after 2 days of resuscitation. After the cells grew to fill the culture dish, passage was performed (generally, cells growing to fill one culture dish may be transferred to five culture dishes), and plasmid transfection can be performed after 4 passages. According to the preferred transfection of the system, PEI as a transfection reagent and a condition of PEI: plasmid (mass ratio) = 2:1 were used for transfection. A mixture of the three-plasmid system and PEI was added to an Opti-MEM medium (Gibco, cat#31985-070), and then the mixture solution was added to the 4th generation HEK293T cells after passages. 6 h after transfection, the medium was replaced with a fresh medium containing 2% FBS, the cells were then cultured for another 72 h, and the supernatant of the HEK293T cells was collected. The collected viral supernatant was concentrated by ultracentrifugation (82200 g, centrifuging at 4°C-8°C for 2 h), and the concentrated virus was filtered with a 0.22 μm filter membrane and sterilized, and then resuspended for later use.

Step 4. Activation of primary T cells

Primary T cells were derived from peripheral blood mononuclear cells (PBMCs) of healthy volunteers. The medium used was a complete medium, ImmunoCult™-XF T Cell Expansion Medium (Stem Cell Technology, cat#10981) + 300 IU/ml IL2 (Cayan, cat# HEILP-0201c). T cells were activated by Dynabeads (Thermo, cat#11141D), Dynabeads: cell = 3: 1. 24 h after activation, the T cells exhibited obviously clustering and enlargement in morphology.

Step 5. Method for gene transduction with lentivirus

48 h after the activation of T cells, the 3E5 cells were cultured in a 24-well culture plate; the prepared lentivirus was added in an amount 3 times the number of the cells, i.e., MOI = 3 (Multiplicity of Infection, a ratio of the number of viruses to the number of cells); and a medium (DMEM medium (+ 10% FBS + 1% P/S)) was supplemented to 500 μL. After 24 h, the medium was supplemented to 1 mL, which was beneficial to cell growth.

Step 6. Candidate sequences of sgRNA

According to the information from relevant websites, sgRNA with sequences of SEQ ID NO: 8 and SEQ ID NO: 9 for editing B2M gene, and sgRNA with a sequence of SEQ ID NO: 10 for editing TRAC gene were screened and designed.

Step 7. Acquisition of Cas9 protein

In this example, Cas9 protein selected was Alt-R s.p. Cas9 Nuclease 3NLS protein from Integrated DNA Technologies (IDT).

Step 8. Preparation of universal-T cells through transformation by electroporation

48 h after transfection of T cells with lentivirus (Jurkat model cell line requires no activation), the cells were collected and washed 3 times with an electroporation buffer, buffer T, T4 (Invitrogen, Lot#1E14211), Opti-MEM (Gibco, cat#31985-070), etc. The cells were resuspended in an electroporation buffer, and the cell density was adjusted to $1 \times 10^8$ cells/mL. In vitro, the above three groups of sgRNAs (150 ng each) were uniformly mixed with 1 μg of Cas9 protein at the same time, and then incubated at room temperature for 10 min. Then the mixture was added into the resuspended cells for electroporation, wherein the total volume was 10 μL in electroporation, and Neon electroporator was used for electroporation. The electroporation conditions were as follows: 1200 v, 10 ms (3 times).

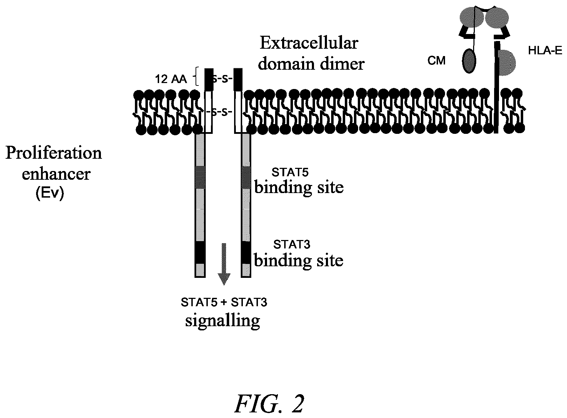

The cells were cultured for 14 days in a complete medium at a density of $1 \times 10^6$ cells/mL, i.e., universal CAR-T cells, in the same medium as above, ImmunoCult™-XF T Cell Expansion Medium (Stem Cell Technology, cat#10981)+ 300 IU/ml IL2 (Cayan, cat# HEILP-0201c). See FIG. 2 for the schematic diagram of co-expression of Ev1-5 proteins and CM protein.

Step 9. Detection of gene transduction efficiency and gene editing efficiency

3 days after electroporation, the editing efficiency in primary T cells was detected. A small number of cells were taken out, washed once with 1 mL of PBS (Gibco, cat# C10010500BT), resuspended in 100 μL of PBS, added with 3 μL of anti-FMC63 antibody (primary antibody, for detecting CAR19), incubated at 4°C for 30 min, washed with 1 mL of PBS, and centrifuged at 300 g for 3 min, and then the supernatant was removed. The cells were resuspended in 100 μL of PBS, added with 0.5 μL of a second antibody for detecting CAR19, 3 μL of an anti-HLA-ABC antibody, 3 μL of an anti-CD3 antibody (for detecting gene editing efficiency) and 3 μL of an anti-HLA-E antibody, uniformly mixed, and incubated at 4°C for 30 min. After washing, the cells were detected by flow cytometry.

**Example 2: Compared with C7R in the prior art, Ev1-5 can effectively improve the lentivirus packaging and production efficiency of CAR-X-CM (X refers to C7R or Ev1-5)**

In the process of preparing and packaging lentiviruses, the length of an inserted gene may affect the lentivirus packaging and production efficiency. Under normal conditions, CAR gene has a length of about 1.5 kb. If a gene sequence greater than 1.5 kb is inserted into the CAR gene, the length is close to the capacity of a lentivirus expression gene (an additional promoter has a length of about 1.5-2 kb, and a terminator WPRE has a length of about 0.5 kb). Although viruses can be packaged and produced, the virus production efficiency may obviously decrease, which is unfavourable for the large-scale preparation of lentiviruses, affecting the yield and subsequent transfection effect. Considering that the length of genes Ev1-5 (786 bp-816 bp) is much smaller than that of C7R (1539 bp), whether Ev1-5 may improve the lentivirus packaging and production efficiency of CAR-X-CM was further explored.

6 viruses, namely lentiviruses containing a target expression plasmid of CJP-P2A-C7R-T2A-CM, CJP-P2A-Ev1-T2A-CM, CJP-P2A-Ev2-T2A-CM, CJP-P2A-Ev3-T2A-CM, CJP-P2A-Ev4-T2A-CM and CJP-P2A-Ev5-T2A-CM, were prepared by the method for preparing lentiviruses in steps 1-3 of example 1. The comparison of virus production efficiency did not require virus purification, but directly detected the titter of the supernatant of lentivirus packaging.

Method for detecting titter of lentiviral supernatant

293T cells were used as target cells to be transfected, and the biological titter of the viral supernatant was calculated by detecting the proportion of CAR molecule positive cells.

1. 293T cells were cultured, and the cultured 293T cells were digested with TrypLE Express Enzyme (1×), phenol red (Gibco, 12605-028) (the medium was a DMEM medium containing 10% FBS). After the cell density was adjusted, the cells were seeded in a 24-well cell culture plate at a seeding amount of 2.5E5 cells per well, with 1.5 ml of the medium per well. The cells were then cultured in a 37°C 5% $CO_2$ incubator for 24 h.

2. According to the number of samples, the number of plate wells was determined, and cells were seeded in another 3 wells at the same time, which were marked as negative control (NC). virus was not seeded in the NC control groups.

3. Before seeding virus, a small number of cells in the 3 NC wells were taken for counting, and the mean was taken, and then multiplied by 1.5 ml to obtain the number of cells during virus infection.

4. 5 virus samples were taken and diluted according to the gradient in the following table, and the diluted samples were added, uniformly mixed and cultured in a 37°C 5% $CO_2$ incubator for 48 h.

| Number | Dilution ratio | Addition volume ($\mu$l) | Volume of corresponding virus stock solution ($\mu$l) |
|---|---|---|---|
| 1 | 1 | 20 | 20 |
| 2 | 2 | 20 | 10 |
| 3 | 20 | 20 | 1 |
| 4 | 200 | 20 | 0.1 |
| 5 | 2000 | 20 | 0.01 |

5. The cells were digested by adding 100 $\mu$l of TrypLE Express Enzyme (1×), phenol red to each well. After leaving the cells to stand for 2 min, 900 $\mu$l of a DMEM medium containing 10% FBS was added for neutralizing. The cells were uniformly mixed by pipetting and blowing, and collected. A small number of cells were taken for counting. According to the counting results, 1E6 cells were placed in a centrifuge tube and centrifuged at 350 g for 5 min, and then the supernatant was removed.

6. Washing: the cells were resuspended by adding 1 ml of PBS to each tube, and centrifuged at 350 g for 5 min, and the supernatant was discarded.

7. Resuspending: the cells were resuspended by adding 100 $\mu$l of PBS to each tube.

8. Adding primary antibody: 1 $\mu$l of primary antibody (Biotin Rabbit Anti-Mouse FMC63scFv) was added to each tube, respectively, and the cells were completely and uniformly mixed, and incubated at 4°C in the dark for 40 min.

9. Washing: 1 ml of PBS was added to each tube after the incubation; the cells were completely and uniformly mixed, and centrifuged at 350 g for 5 min; and the supernatant was discarded. The above operations were repeated once.

10. Adding second antibody: the cells were resuspended by adding 100 $\mu$l of PBS to each tube, then 0.5 $\mu$l of second antibody (streptavidin PE) was added to each tube, and the cells were incubated at 4°C in the dark for 40 min.

11. Washing: 1 ml of PBS was added to each tube after the incubation; the cells were completely and uniformly mixed, and centrifuged at 350 g for 5 min; and the supernatant was discarded. The above operations were repeated 2 times, and then the cells were resuspended in 100 $\mu$l of PBS.

12. The cells in the NC wells were divided into 3 groups. The cells in the first group were not stained. After being digested and collected, the cells were washed once with PBS, and then resuspended in 100 $\mu$l of PBS, which were marked as NC without staining. The cells in the second group were not added with primary antibody, but only second antibody, which were used for excluding specific staining and marked as NC PE-strep. The cells in third group were stained normally, which were marked as NC with staining.

13. On-machine detection: first, samples of NC without staining were collected; voltage regulation and gate setting were performed; then substitute samples were sequentially collected; and the CAR expression efficiency results were obtained.

14. The formula for calculating lentivirus titter (TU/ml) was as follows:

$$TU/ml = (C \times N \times 0.01 \times 1000 \times D)/V$$

C = CAR expression efficiency (%); N = Number of cells during infection (about 5E5); D = Dilution ratio of virus sample; V = Volume of added diluted virus ($\mu$l).

**Example 3: Compared with C7R in the prior art, Ev1-5 can effectively improve the transfection efficiency of universal CAR-T cells**

In order to perform parallel comparison on the transfection efficiency of universal CAR-T cells in primary T cells, the lentiviruses in each group transfected primary T cells according to the same MOI after purification. The method for preparing lentiviruses was the same as that in step 3 of example 1, and 5 viruses of example 2 were prepared.

Transfection according to the same MOI can ensure that the difference in transfection results is not caused by the virus production efficiency of supernatants.

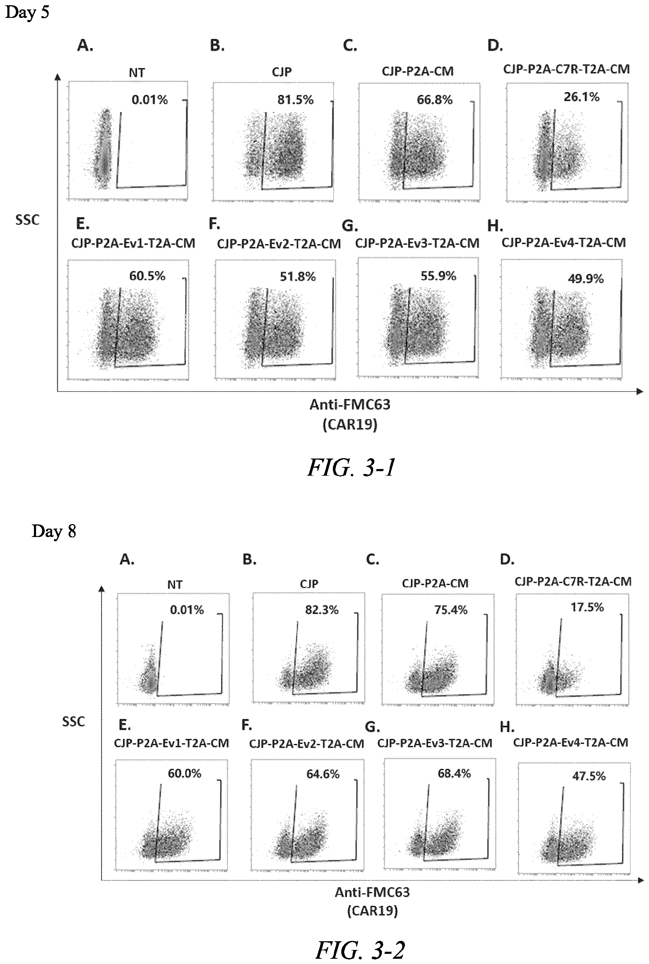

48 h after activation of PBMC, the lentiviruses in each group transfected primary T cells under a condition of MOI = 3, and the CAR expression efficiency was detected on day 3 after transfection (i.e., day 5 after activation, FIG. 3-1). As shown in FIG. 3-1, under the same transfection condition, the group expressing only CJP had an efficiency of 81.5% (B in FIG. 3-1), the group expressing CJP-P2A-CM had an efficiency of 66.8% (C in FIG. 3-1), and the group expressing CJP-P2A-C7R-T2A-CM had a significantly decreased efficiency of only 26.1% (D in FIG. 3-1), indicating that the introduction of C7R had an adverse effect on the transfection efficiency and expression efficiency of CAR. In contrast, when C7R was replaced with Ev1-5, the expression efficiency in each group was significantly increased, ranging from 50% to 60%, which was similar to that of CJP-P2A-CM.

**Example 4: Compared with C7R in the prior art, Ev1-5 can effectively improve the stability of CAR expression in universal CAR-T cells**

CAR is a protein expressed on cell membrane. After transcription and translation, CAR gene is transported to the surface of the cell membrane through vesicles. The expression of CAR is a dynamic process of cell membrane displayendocytosis. The stability of CAR expression is essential for the function of CAR-T cells. If the expression of CAR on the cell membrane is blocked due to factors such as expression efficiency and endocytosis speed, the activity of CAR-T cells may be seriously affected. Therefore, the effects of C7R and Ev1-5 on the stability of CAR expression were further compared.

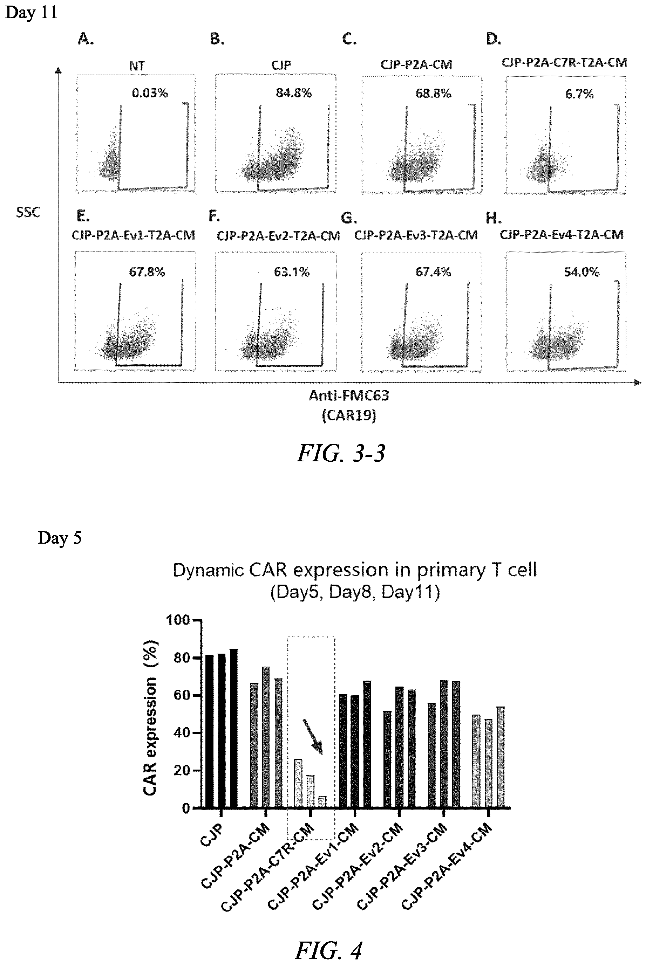

The experimental method was the same as that of example 3. On day 5 after the experiment, the dynamic expression change of CAR during the preparation and proliferation of universal CAR-T cells was continuously tracked. As shown in FIG. 3-1, FIG. 3-2 and FIG. 3-3, on day 5, day 8 and day 11, the proportion of CAR positive cells in each group were detected, wherein the proportion of CAR positive cells in the CJP-P2A-C7R-T2A-CM group gradually decreased to 26.1%, 17.5% and 6.7%, respectively, and the stable expression proportion were maintained in the other groups compared with the initial transfection efficiency. FIG. 4 showed the statistical data of each group. The results showed that the introduction of C7R made the expression efficiency of CAR in primary T cells unstable, and the expression tended to decrease with the culture time, while the introduction of Ev1-5 can stabilize the stable expression of CAR molecules.

**Example 5: Ev1-5 can effectively activate STAT5 and (or) STAT3 signalling**

STATS and STAT3 are important signalling proteins in the IL-7 and IL-2 cytokine signalling pathways. After responding to the signal, pSTAT5 and pSTAT3 may activate a series of downstream genes, which are beneficial to the survival and proliferation of T cells. By screening extracellular domains, although the size of Ev1-5 molecule is much smaller than that of C7R, Ev1-5 can form a dimer and efficiently activate STATS and STAT3 signalling. The expression of pSTAT5 and pSTAT3 is used as the detection of cells in each group.

Method for detecting extracellular CAR and intracellular pSTAT5/3 expression by flow cytometry

1. Cells in a positive control group (T cells expressing CJP) were treated with 600 IU IL-2 for 30 min in advance to activate the expression of pSTAT5 and pSTAT3. Cells in other groups were not treated.

2. Extracellular CAR staining: 1 mL of cells in each group were resuspended in PBS at a density of 5E6 cells/mL, and with reference to example 1, extracellular CAR was stained by adding an antibody for detecting CAR.

3. Cell immobilization: the cells were resuspended in 250 uL of PBS, added with the same volume of IC immobilization solution (BioLegend, 420801), and immobilized by vortex mixing.

4. Samples were incubated at room temperature in the dark for 30 min.

5. The samples were centrifuged at 500 g at room temperature for 5 min, and the supernatant was discarded.

6. The cell precipitate was resuspended, and added with 1 mL of precooled 90%-100% methanol (BBI life science, MT1617). The cells were subjected to vortex mixing, and incubated at 2°C-8°C for 30 min.

7. Methanol was washed out. The cells were washed with an excessive volume (10 mL in this example) of PBS.

8. The cells were centrifuged at 500 g at room temperature for 5 min, and the supernatant was discarded.

9. The cells were resuspended in a PBS solution at a density of 1E7 cells/mL, and 100 μL of the cells was distributed to a flow cytometry tube.

10. Antibody Anti-pSTAT5 (pY694) (BD Pharmingen, 612567) or Anti-pSTAT3 (pY705) (BD Pharmingen, 612569) was added, and the cells were incubated at room temperature for 30-60 minutes in the dark.

11. 2 mL of a flow cytometry staining solution was added; the cells were centrifuged at 500 g at room temperature for 5 min; and the supernatant was discarded.

12. Step 11 was repeated, and the cells were washed twice.

13. The stained cells were suspended in 200 uL of PBS, and analysed by flow cytometry.

Preparation of cells expressing CJP-P2A-C7R-T2A-CM and CJP-P2A-(Ev1-5)-T2A-CM

The purpose of this example was to detect the expression of pSTAT5 and pSTAT3 in the cells in each group. In order to reduce the background interference in detection, a Jurkat cell line was used to prepare CAR-T cells in each group. Jurkat cells are common model cells for studying T cell signal transduction. 1E6 Jurkat cells were taken and resuspended to 1 mL, plated on a 24-well culture plate, and transfected with the lentiviruses in each group under a condition of MOI = 0.5, and the medium was changed after 24 h. 3 days after culture, the expression of CAR can be detected, and was used to detect the expression of pSTAT5 and pSTAT3. In particular, due to the low expression efficiency of CJP-P2A-C7R-T2A-CM, the transfection condition of MOI = 1 was used in this group under the same condition.

Analysis of expression of pSTAT5 and pSTAT3

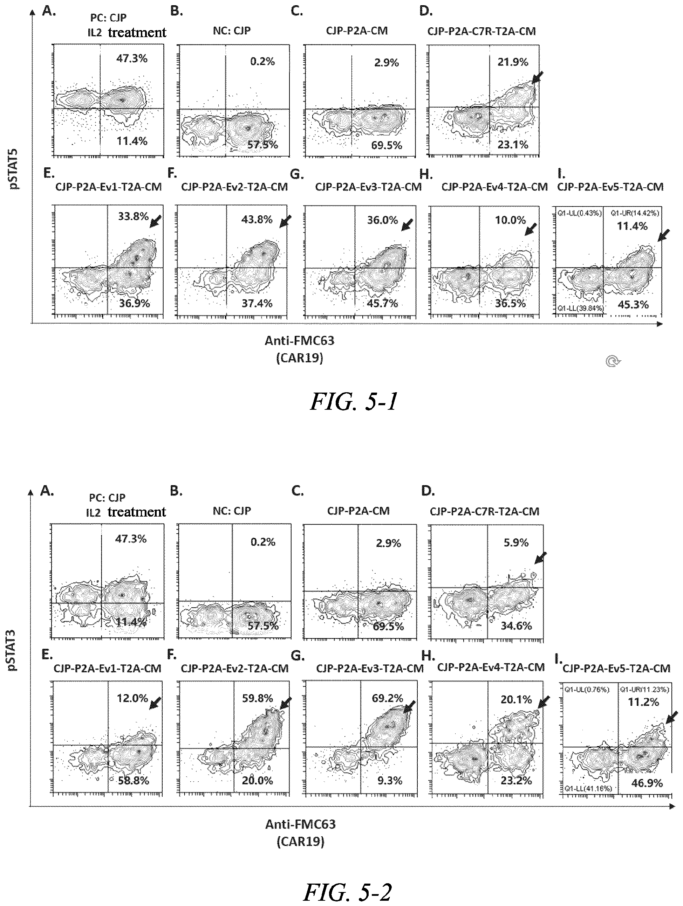

For pSTAT5, as shown in FIG. 5-1, most cells in the positive control group expressed pSTAT5 after activation by IL-2, and this expression was independent of the expression of CAR (A in FIG. 5-1); for the C7R group in the prior art (D in FIG. 5-1), the expression of pSTAT5 can be detected in some CAR positive cells, and the expression efficiency was 21.9%, of which the CAR positive cells expressing pSTAT5 accounted for about 46.7%; and Ev1-5 groups each also had an effective expression of pSTAT5, and the CAR positive cells expressing pSTAT5 accounted for 33.8%, 43.8%, 36.0%, 10.0% and 11.4% of the total cells, respectively, of which except Ev4 group and Ev5 group, the proportion of the CAR positive cells expressing pSTAT5 in other groups was similar to that of C7R, ranging from 45% to 55%.

For pSTAT3, as shown in FIG. 5-2, about half of the cells in the positive control group expressed pSTAT3 after activation by IL-2, and this expression was independent of the expression of CAR (A in FIG. 5-2); for the C7R group in the prior art (D in FIG. 5-2), only weak expression of pSTAT3 was detected, about 5.9%, indicating that C7R only weakly activated STAT3 signalling pathway; and the expression efficiency of pSTAT3 in Ev1-5 groups increased to varying degrees, indicating that Ev1-5 can activate both STAT5 and STAT3 signalling pathways, and the intensity varied with different molecules.

**Example 6: Ev1-5 can effectively improve the in vitro proliferation ability of universal CAR-T cells**

Universal CAR-T cells (U represents universal T cells) in each group were prepared according to example 1, and cultured until day 14. 1E5 CAR positive cells were taken from the cells in each group and used for proliferation experiments stimulated by tumour cells in vitro. Tumour cells were CD19 positive Raji cells, and the Raji cells stably expressed GFP fluorescent protein, which was used to distinguish CAR-T cells from the tumour cells. The medium was X-VIVO™ 15 Medium (Lonza, 04-418Q, IL-2-free), CAR positive cells: Raji cells = 1 : 1. The cells were seeded in a 24-well cell culture plate, and the medium was supplemented to 1 mL. Every 2-3 days, 100 ul of the sample was taken, and CAR positive cells were detected by flow cytometry according to the method of example 1, that is, the cells were washed once by adding 1 mL of PBS (Gibco, cat# C10010500BT) to 100 uL of the detection solution, resuspended in 100 μL of PBS, sequentially added with 3 μL of anti-FMC63 antibody (primary antibody, for detecting CAR19) and 0.5 μL of second antibody, uniformly mixed, and incubated at 4°C for 30 min. After washing, the cells were detected by flow cytometry. On day 7, the cells were repeatedly stimulated by tumour cells; 1E5 Raji cells were added to each group; the medium was supplemented to 1 mL; and the above experiment was repeated for subsequent detection.

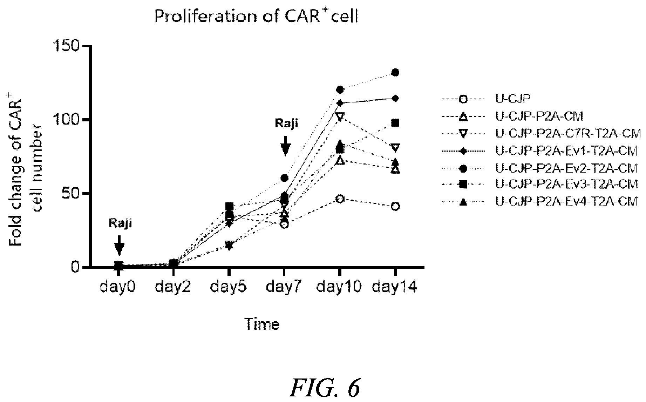

As shown in FIG. 6, there was little difference in the proliferation ability of CAR positive cells in each group after the first stimulation by tumour cells. After the repeated stimulation on day 7, the proliferation in U-CJP group was limited and in a slow state, and increased to about 40-50 times between 10-14 days; in contrast, after repeated stimulation on day 7, the proliferation of U-CJP-P2A-CM cells with chimeric molecule (CM) increased to a certain extent, by about 70 times; the introduction of C7R can obviously improve the proliferation ability of cells, and after the repeated stimulation on day 7, the proliferation can increase to 80-100 times; and in contrast, Ev1-4 all can significantly improve the proliferation ability of cells, and the increase in the proliferation varied from 70 times to 130 times on day 14. In particular, the proliferation in the two groups, i.e., U-CJP-P2A-Ev1-T2A-CM and U-CJP-P2A-Ev2-T2A-CM, were the strongest, and increased to 110 times or more, and the proliferation ability was still maintained from day 10 to day 14. In summary, the experimental data showed that Ev1-4 can effectively improve the in vitro proliferation ability of universal CAR-T cells, and Ev1 and Ev2 performed better, and were selected to continue the subsequent anti-rejection functional experiments.

U-CJP represents universal T cells expressing CAR molecule CJP; U-CJP-P2A-CM represents universal CAR-T cells expressing CAR molecule CJP and chimeric molecule (CM); U-CJP-P2A-Ev1-T2A-CM represents universal CAR-T cells expressing CAR molecule CJP, proliferation enhancer Ev1 and chimeric molecule (CM).

**Example 7: In an immunological rejection environment, universal CAR-T cells expressing Ev molecule have stronger proliferation ability than universal CAR-T cells expressing C7R molecule**

Preparation of universal CAR-T cells and detection of cell phenotype

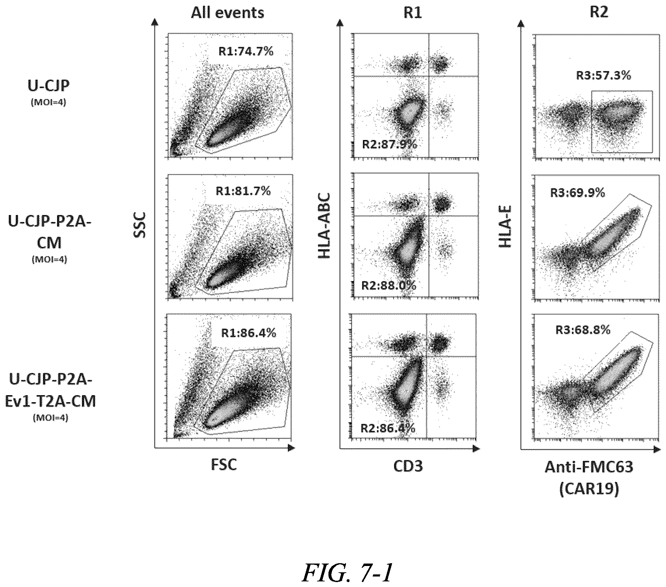

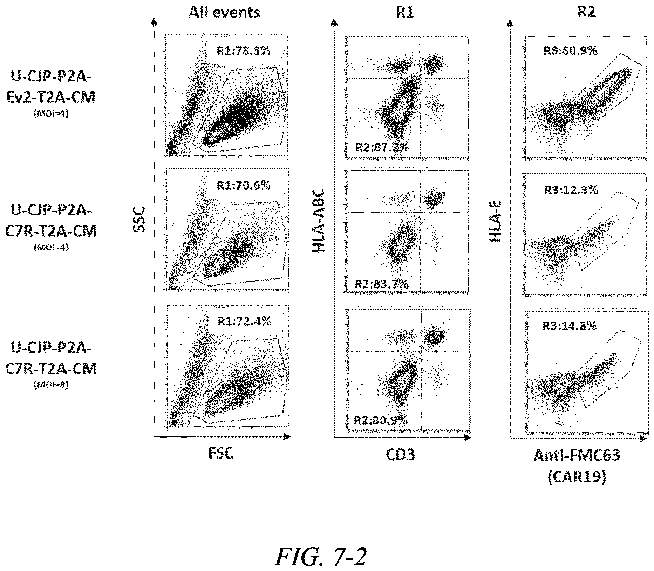

The preparation and flow cytometry detection of universal CAR-T cells in each group were the same as those in example 1. Considering that the transfection efficiency of C7R was relatively low and unstable under the same condition, in order to complete functional experiments, MOI was increased. In this example, MOI = 4 was used, and another C7R group under a condition of MOI = 8 was added. The results were as shown in FIG. 7-1 and FIG. 7-2. All groups achieved high gene editing efficiency, that is, the proportion of CD3/HLA-ABC double negative cells was greater than 80%; except for the C7R group, the proportion of CAR positive cells in the universal CAR-T cells in each group was 50%-70%, and CAR and HLA-E (HLA-E being used for detecting the expression of CM) had efficient co-expression; and as shown in FIG. 7-2, the expression was only 12.3% in U-CJP-P2A-C7R-T2A-CM under the same virus transfection condition (MOI = 4), and the expression was only 14.8% even under the condition of MOI = 8, which were similar to the results of example 3, indicating that C7R significantly affected the expression of CAR. For subsequent functional evaluation, an experiment strategy in which the number of CAR positive cells in each group was the same was used. Therefore, the proportion of CAR positive cells being 14.8% did not affect the subsequent functional evaluation.

Detection of HLA-E expression abundance of universal CAR-T cells

HLA-E can effectively inhibit the cytotoxic response of NK cells and T cells by interacting with NKG2A/CD94, and the expression of HLA-E by universal CAR-T cells may effectively alleviate the killing by NK cells and allogeneic PBMC. Therefore, the expression of higher abundance of HLA-E molecules by universal CAR-T cells may enhance the resistance of universal CAR-T cells to immunological rejection.

About 1E5 cells were taken out, washed once with 1 mL of PBS (Gibco, cat# C10010500BT), resuspended in 100 μL of PBS, added with 3 μL of anti-FMC63 antibody (primary antibody, for detecting CAR19), incubated at 4°C for 30 min, washed with 1 mL of PBS, and centrifuged at 300 g for 3 min, and then the supernatant was removed. The cells were resuspended in 100 μL of PBS, added with 0.5 μL of a second antibody for detecting CAR19, 3 μL of an anti-HLA-ABC antibody, 3 μL of an anti-CD3 antibody (for detecting gene editing efficiency) and 3 μL of an anti-HLA-E antibody, uniformly mixed, and incubated at 4°C for 30 min. After washing, the cells were detected by flow cytometry. Flow cytometry analysis was performed, with the detection method as shown in example 4, that is, the mean fluorescence intensity of the HLA-E expression abundance in HLA-ABC cells, CD3 double negative cells and CAR positive cells were analysed.

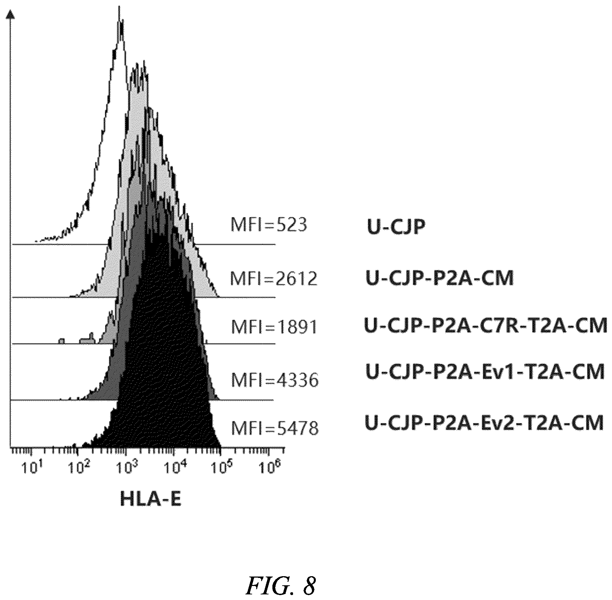

As shown in FIG. 8, the MFI of HLA-E expression in U-CJP cells was 523, which was a negative background expression; and the MFI of the expression in the U-CJP-P2A-CM group expressing heterogeneous chimeric molecule (CM) was 2612, with the expression abundance increasing to 5 times. After the introduction of C7R, the MFI of HLA-E expression in U-CJP-P2A-C7R-T2A-CM was 1891, which was 3.6 times the MFI of the background expression. The MFI of HLA-E expression in the U-CJP-P2A-Ev1-T2A-CM group and the U-CJP-P2A-Ev2-T2A-CM group were 4335 and 5478, respectively, which were 8.3 times and 10.5 times the MFI of the background expression, respectively. The introduction of Ev1/2 significantly increased the expression abundance of HLA-E, suggesting that universal CAR-T cells in this group may have stronger ability to resist rejection and killing.

Establishment of co-culture experiment of universal CAR-T cells and allogeneic PBMC cells.

This experiment is to scientifically simulate the in vivo environment. Because immune cells in the body are complex and include not only NK cells and target cells, it is more realistic to establish a true allogeneic PBMC environment to approach the in vivo environment. The proliferation ability of edited CAR-T cells in an allogeneic PBMC environment is detected, and in the environment, allogeneic immunological rejection (not only from NK cells) is present, and target cells (tumour Raji cells) are also present.

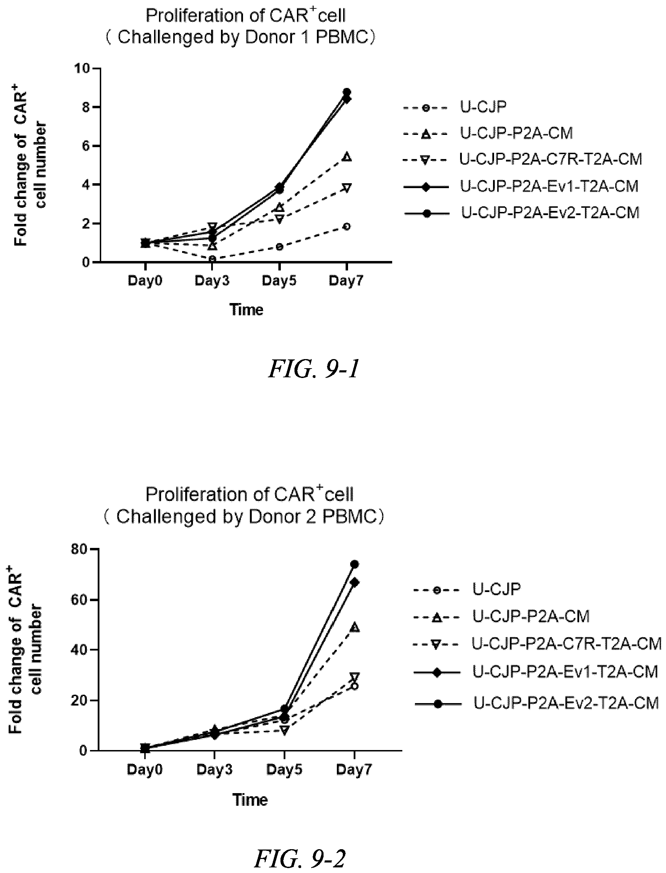

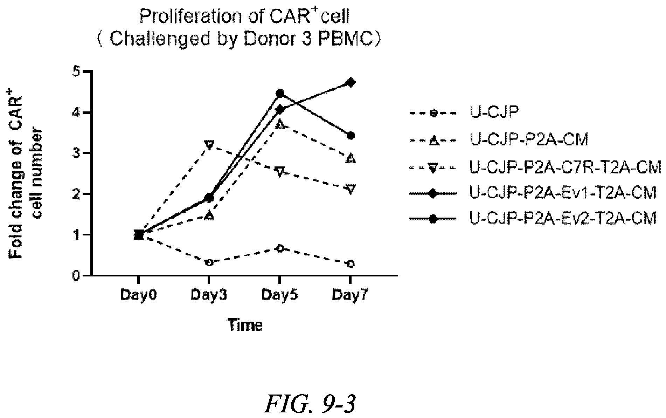

Cells were co-cultured, wherein universal CAR-T cells: tumour Raji cells: allogeneic PBMC = 1: 5: 20. The number of CAR positive cells in universal CAR-T cells was 5E4 cell/ml, and other cells were extrapolated proportionally. The cells were cultured in a 24-well culture plate at 37°C. The number of CAR positive cells in different groups was recorded at different time points (FIG. 9-1 to FIG. 9-3).

Before co-culture of allogeneic PBMC and Raji cells, staining was performed with Dye eFluor™ 670 to distinguish universal CAR-T cells: the cell density was adjusted to 1E7 cells/ml. e670 dye with a final concentration of 10 μM was added, and the cells were incubated at room temperature in the dark for 5 min, washed with a medium three times, and then used in experiments.

In this example, the allogeneic PBMCs were from three different healthy donors, and the data of donors 1, 2 and 3 were as shown in FIG. 9-1, FIG. 9-2 and FIG. 9-3 respectively. Donor 1 had the same source of PBMCs as the prepared universal CAR-T cells, i.e., autologous PBMCs, and the others were allogeneic PBMCs. As shown in FIG. 9-1 to FIG. 9-3, in the 3 immunological rejection PBMC environments, the proliferation of U-CJP was the weakest, suggesting that this group had the poorest resistance to rejection; and the proliferation ability of U-CJP-P2A-CM was better than that of U-CJP, but the introduction of C7R into U-CJP-P2A-C7R-T2A-CM failed to show the proliferation advantage compared to U-CJP-P2A-CM, which can be related to the low expression efficiency and unstable expression of CAR or the low HLA-E expression abundance. In particular, in the three groups of rejection experiments, U-CJP-P2A-Ev1-T2A-CM and U-CJP-P2A-Ev1-T2A-CM both showed obvious proliferation advantages, indicating that the combined expression of Ev and CM can effectively improve the proliferation ability and resistance to rejection and killing of universal CAR-T cells.

## Claims

1.  A proliferation enhancer, comprising the following components: a) one or more cytokine receptor intracellular domains capable of initiating a cellular STATS and/or STAT3 signalling pathway; b) a transmembrane domain; c) one or more extracellular domains that are extracellular domains of DAP12 or variants thereof; wherein the transmembrane domain and the extracellular domain comprise a structure that promotes the homodimerization of the proliferation enhancer.

2.  The proliferation enhancer of claim 1, wherein the receptor intracellular domain is from an IL-7 cytokine receptor α, an IL-21 cytokine receptor α, an IL-23 cytokine receptor α, an IL-12 cytokine receptor α, CD122, or a combination thereof.

3.  The proliferation enhancer of claim 1, wherein the receptor intracellular domain is a sequence of amino acids at positions 47-241 of SEQ ID NO: 3, amino acids at positions 47-245 of SEQ ID NO: 4, amino acids at positions 47-251 of SEQ ID NO: 5 or amino acids at positions 47-266 of SEQ ID NO: 6.

4.  The proliferation enhancer of any one of the preceding claims, wherein the transmembrane domain is an endogenous transmembrane domain of component a) one or more cytokine receptor intracellular domains, or a variant of the endogenous transmembrane domain.

5.  The proliferation enhancer of any one of the preceding claims, wherein the transmembrane domain is a transmembrane domain of an IL-7 cytokine receptor α or a variant thereof, or the transmembrane domain is a transmembrane domain of DAP12 or a variant thereof.

6.  The proliferation enhancer of any one of the preceding claims, wherein the transmembrane domain comprises at least one cysteine.

7.  The proliferation enhancer of any one of the preceding claims, wherein the transmembrane domain is a sequence having a length of 21-33 amino acids.

8.  The proliferation enhancer of claim 7, wherein the transmembrane domain is a sequence of amino acids at positions 19-46 of SEQ ID NO: 3, or the transmembrane domain is SEQ ID NO: 11, or the transmembrane domain is a sequence of amino acids at positions 20-40 of SEQ ID NO: 15.

9.  The proliferation enhancer of any one of the preceding claims, wherein the extracellular domain is an extracellular domain of DAP12 having a sequence as shown in amino acids at positions 7-18 of SEQ ID NO: 3, or a polypeptide sequence comprising an extracellular domain of DAP12, or a variant of an extracellular domain of DAP12 which is derived by performing substitution, deletion or addition of one or several amino acids on an extracellular domain of DAP12.

10. The proliferation enhancer of claim 9, wherein the extracellular domain has a length of not more than 130 amino acids; preferably not more than 120 amino acids; preferably not more than 40 amino acids; preferably not more than 20 amino acids; preferably, the extracellular domain has a length of 10-40 amino acids or 12-20 amino acids.

11. A nucleic acid sequence expressing the proliferation enhancer of any one of the preceding claims.

12. A genetically engineered cell, **characterized in that** on the surface of the cell, the proliferation enhancer of any one of claims 1-10 is expressed.

13. The cell of claim 12, wherein the cell is an immune cell, and further the immune cell is preferably a lymphocyte or a macrophage.

14. The cell of claim 13, wherein the lymphocyte is a T cell, an NKT cell, a γδT cell, a mucosa associated invariant T cell, an NK cell or a B cell.

15. The cell of any one of claims 12-14, wherein the surface of the cell lacks at least one endogenously expressed MHC molecule or MHC molecule analogue.

16. The cell of any one of claims 12-15, wherein at least one gene encoding a component of an endogenous T cell receptor (TCR) in the cell is inactivated.

17. The cell of any one of claims 12-16, wherein the T cell is a CAR-T cell or a TCR-T cell.

18. The cell of any one of claims 12-17, wherein the cell has a nucleic acid sequence expressing a chimeric single chain molecule, and the chimeric single chain molecule comprises: (a) a presenting peptide fragment, (b) a B2M protein, and (c) a linker sequence for linking the aforementioned fragments (a) and (b); wherein, the chimeric single chain molecule forms a complex on the cell membrane with a heavy chain molecule of MHC or an MHC analogue; and the presenting peptide fragment is a polypeptide sequence of 5-30 amino acids.

19. The cell of claim 18, wherein the chimeric single chain molecule sequentially comprises, from the N-terminus to the C-terminus, presenting peptide fragment-linker sequence-B2M protein, or B2M protein-linker sequence-presenting peptide fragment.

20. The cell of claim 18 or 19, wherein the heavy chain molecule of MHC is a heavy chain molecule endogenously expressed in a cell and selected from a classical class I MHC molecule, preferably a heavy chain molecule of HLA-A, HLA-B or HLA-C; or the heavy chain molecule of MHC is a heavy chain molecule endogenously expressed in a cell and selected from a non-classical class I MHC molecule, preferably a heavy chain molecule of HLA-E, HLA-F or HLA-G; or the heavy chain molecule of MHC is a heavy chain molecule endogenously expressed in a cell and selected from other non-classical class I MHC molecules, preferably a heavy chain molecule of CD1 or MR1 or LTL18.

21. The cell of any one of claims 18-20, wherein the presenting peptide fragment is derived from a signal peptide of a class I MHC molecule.

22. The cell of any one of claims 17-21, wherein the cell lacks an endogenously expressed MHC molecule, and lacks endogenously expressed T cell receptor.

23. The cell of claim 22, wherein the chimeric single chain molecule expressed by the cell forms a complex on the cell membrane with a heavy chain molecule of MHC or an MHC analogue.

24. Use of the cell of any one of claims 12-23 in the preparation of a drug for treating a cancer.

25. A method for treating a cancer, comprising: administering to an individual the genetically engineered cell of claims 12-23.

26. The use of claim 24 and the method of claim 25, wherein the cancer is breast cancer, prostate cancer, lung cancer, brain cancer, colon cancer, head and neck cancer, skin cancer, ovarian cancer, endometrial cancer, cervical cancer, kidney cancer, lung cancer, gastric cancer, small intestine cancer, liver cancer, pancreatic cancer, gallbladder cancer, carcinoma of bile duct, oesophageal cancer, salivary adenocarcinoma or thyroid cancer.

*FIG. 1*

Extracellular
domain dimer

CM

HLA-E

12 AA

S-S-

S-S-

Proliferation
enhancer
(Ev)

STAT5
binding site

STAT3
binding site

STAT5 + STAT3
signalling

*FIG. 2*

Day 5

FIG. 3-1

Day 8

FIG. 3-2

Day 11

SSC

Anti-FMC63
(CAR19)

*FIG. 3-3*

Day 5

Dynamic CAR expression in primary T cell
(Day5, Day8, Day11)

*FIG. 4*

*FIG. 5-1*

*FIG. 5-2*

FIG. 6

*FIG. 7-1*

*FIG. 7-2*

*FIG. 8*

Proliferation of CAR$^+$cell
( Challenged by Donor 1 PBMC)

- U-CJP
- U-CJP-P2A-CM
- U-CJP-P2A-C7R-T2A-CM
- U-CJP-P2A-Ev1-T2A-CM
- U-CJP-P2A-Ev2-T2A-CM

*FIG. 9-1*

Proliferation of CAR$^+$cell
( Challenged by Donor 2 PBMC)

- U-CJP
- U-CJP-P2A-CM
- U-CJP-P2A-C7R-T2A-CM
- U-CJP-P2A-Ev1-T2A-CM
- U-CJP-P2A-Ev2-T2A-CM

*FIG. 9-2*

FIG. 9-3

## INTERNATIONAL SEARCH REPORT

| International application No. |
|---|
| **PCT/CN2022/075873** |

**A. CLASSIFICATION OF SUBJECT MATTER**

C07K 14/715(2006.01)i; A61K 35/17(2015.01)i; C12N 5/10(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K A61K C12N A61P

Documentation searched other than minimum documentation to the extent that such documents are included in the fields searched

Electronic data base consulted during the international search (name of data base and, where practicable, search terms used)

CNABS, CNTXT, SIPOABS, DWPI, VEN, WOTXT, USTXT, EPTXT, CNKI, 万方数据库, WANFANG DATABASE, baidu 学术, BAIDU SCHOLAR, PUBMED, ELSEVIER, SPRINGER, ISI web of knowledge, sciencedirect, NCBI, Genbank, EMBL, STN, 中国专利生物序列检索系统, Chinese Patent Biological Sequence Retrieval System: 细胞因子受体, IL-7, IL-21, IL-23, IL-12, CD122, IL-7R, IL-21R, IL-23R, IL-12R, CD122R, cytokine receptor, DAP12, TYRO蛋白酪氨酸激酶结合蛋白, TYROBP, KARAP, PLOSL, 胞内, 跨膜, 胞外, intracellular, transmembrane, extracellular, 二聚化, dimerization, 克睿基因, GURE GENETICS, 发明人

**C. DOCUMENTS CONSIDERED TO BE RELEVANT**

| Category* | Citation of document, with indication, where appropriate, of the relevant passages | Relevant to claim No. |
|---|---|---|
| A | CN 109952309 A (BAYLOR COLLEGE OF MEDICINE) 28 June 2019 (2019-06-28) entire document | 1-26 |
| A | CN 109694854 A (GRACELL BIOTECHNOLOGY (SHANGHAI) CO., LTD.) 30 April 2019 (2019-04-30) entire document | 1-26 |
| A | WO 2020/163634 A1 (SEATTLE CHILDREN'S HOSPITAL DBA SEATTLE CHILDREN'S RESEARCH INSTITUTE) 13 August 2020 (2020-08-13) entire document | 1-26 |
| A | WO 2020/097164 A1 (WASHINGTON UNIVERSITY) 14 May 2020 (2020-05-14) entire document | 1-26 |
| A | WANG, E. et al. "Generation of Potent T-cell Immunotherapy for Cancer Using DAP12-Based, Multichain, Chimeric Immunoreceptors" *Cancer Immunology Research*, Vol. 3, No. 7, 04 May 2015 (2015-05-04), pp. 815-826 | 1-26 |

☑ Further documents are listed in the continuation of Box C. ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **25 April 2022** | **07 May 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/075873** |

**C.      DOCUMENTS CONSIDERED TO BE RELEVANT**

| Category* | Citation of document, with indication, where appropriate, of the relevant passages | Relevant to claim No. |
| --- | --- | --- |
| A | ZABEL, M. et al. "The making and function of CAR cells"<br>*Immunology Letters,* Vol. 212, 07 June 2019 (2019-06-07),<br>     pp. 53-69 | 1-26 |
| A | CHEN, B. et al. "TREM1/Dap12-based CAR-T cells show potent antitumor activity"<br>*Immunotherapy,* Vol. 11, No. 12, 02 July 2019 (2019-07-02),<br>     pp. 1043-1055 | 1-26 |
| A | SHUM, T. et al. "Constitutive signaling from an engineered IL7 receptor promotes durable<br>tumor elimination by tumor-redirected T cells"<br>*Cancer Discovery,* Vol. 7, No. 11, 22 August 2017 (2017-08-22),<br>     pp. 1238-1247 | 1-26 |

Form PCT/ISA/210 (second sheet) (January 2015)

**INTERNATIONAL SEARCH REPORT**

International application No.

**PCT/CN2022/075873**

| Box No. I | Nucleotide and/or amino acid sequence(s) (Continuation of item 1.c of the first sheet) |
|---|---|

1.  With regard to any nucleotide and/or amino acid sequence disclosed in the international application, the international search was carried out on the basis of a sequence listing:

    a.  ☑  forming part of the international application as filed:

          ☑  in the form of an Annex C/ST.25 text file.

          ☐  on paper or in the form of an image file.

    b.  ☐  furnished together with the international application under PCT Rule 13*ter*.1(a) for the purposes of international search only in the form of an Annex C/ST.25 text file.

    c.  ☐  furnished subsequent to the international filing date for the purposes of international search only:

          ☐  in the form of an Annex C/ST.25 text file (Rule 13*ter*.1(a)).

          ☐  on paper or in the form of an image file (Rule 13*ter*.1(b) and Administrative Instructions, Section 713).

2.  ☐  In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## INTERNATIONAL SEARCH REPORT

| International application No. |
|---|
| **PCT/CN2022/075873** |

**Box No. II     Observations where certain claims were found unsearchable (Continuation of item 2 of first sheet)**

This international search report has not been established in respect of certain claims under Article 17(2)(a) for the following reasons:

1. ☑ Claims Nos.: **25，26**（部分）
   because they relate to subject matter not required to be searched by this Authority, namely:

   [1]   Claims 25 and 26 (in part) relate to a method for treating cancer, i.e., claims 25 and 26 (in part) relate to the subject matter defined in PCT Rule 39.1 that does not warrant a search conducted by the international searching authority: (iv) a method for treatment of a human body or animal body by surgery or therapy and a diagnostic method. The international search is carried out on the basis of a use of a genetically engineered cell in the preparation of a drug for treating cancer.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

## INTERNATIONAL SEARCH REPORT
### Information on patent family members

International application No.

**PCT/CN2022/075873**

| Patent document cited in search report | | | Publication date (day/month/year) | Patent family member(s) | | | Publication date (day/month/year) |
|---|---|---|---|---|---|---|---|
| CN | 109952309 | A | 28 June 2019 | CA | 3034873 | A1 | 01 March 2018 |
| | | | | KR | 20190039597 | A | 12 April 2019 |
| | | | | US | 2019183936 | A1 | 20 June 2019 |
| | | | | AU | 2017317022 | A1 | 14 March 2019 |
| | | | | WO | 2018038945 | A1 | 01 March 2018 |
| | | | | SG | 11201901642 X | A | 28 March 2019 |
| | | | | EP | 3504223 | A1 | 03 July 2019 |
| | | | | JP | 2019528078 | A | 10 October 2019 |
| CN | 109694854 | A | 30 April 2019 | None | | | |
| WO | 2020/163634 | A1 | 13 August 2020 | AU | 2020219246 | A1 | 19 August 2021 |
| | | | | KR | 20210141479 | A | 23 November 2021 |
| | | | | CN | 113660942 | A | 16 November 2021 |
| | | | | US | 2022096549 | A1 | 31 March 2022 |
| | | | | EP | 3920952 | A1 | 15 December 2021 |
| | | | | IL | 285230 | D0 | 30 September 2021 |
| | | | | CA | 3129229 | A1 | 13 August 2020 |
| | | | | JP | 2022519704 | A | 24 March 2022 |
| WO | 2020/097164 | A1 | 14 May 2020 | SG | 11202104287 R | A | 28 May 2021 |
| | | | | IL | 282850 | D0 | 30 June 2021 |
| | | | | KR | 20210090220 | A | 19 July 2021 |
| | | | | CN | 113423409 | A | 21 September 2021 |
| | | | | JP | 2022512922 | A | 07 February 2022 |
| | | | | CA | 3117206 | A1 | 14 May 2020 |
| | | | | EP | 3876961 | A1 | 15 September 2021 |
| | | | | US | 2022073585 | A1 | 10 March 2022 |
| | | | | AU | 2019377461 | A1 | 27 May 2021 |

Form PCT/ISA/210 (patent family annex) (January 2015)

**EP 4 293 041 A1**

### Patent documents cited in the description

- CN 109952309
- CN 106103475 A
- CN 201780065147

### Non-patent literature cited in the description

- **NEEDLEMAN ; WUNSCH.** *J. Mol. Biol.,* 1970, vol. 48, 443-453
- **RICE et al.** EMBOSS: The European Molecular Biology Open Software Suite. *Trends in Genetics,* 2000, vol. 16, 276-277
- **CELIK et al.** *Immunogenetics,* 2016, vol. 68, 29-41
- **HANNOUN et al.** *Immunology Letters,* 2018, vol. 202, 65-72
- **RÖLLE et al.** *Cell Rep,* 2018, vol. 14 (8), 1967-1976
- **RÖLLE et al.** *Front Immunol,* 2018, vol. 9, 2410